# EUROPEAN PATENT SPECIFICATION

(11) **EP 3 795 999 B1**
(45) Date of publication and mention of the grant of the patent: **29.07.2026**
(21) Application number: 20190583.3
(22) Date of filing: 18.06.2015
(51) Int. Cl.: G01N 33/533, G01N 33/542, G01N 21/64, G01N 33/68

(54) **A METHOD OF DETERMINING THE ABUNDANCE OF A TARGET MOLECULE IN A SAMPLE**
VERFAHREN ZUR BESTIMMUNG DER MENGE EINES ZIELMOLEKÜLS IN EINER PROBE
PROCÉDÉ PERMETTANT DE DÉTERMINER L'ABONDANCE D'UNE MOLÉCULE CIBLE DANS UN ÉCHANTILLON

(30) Priority: 15.07.2014 EP 14177005
(43) Date of publication of application: 24.03.2021
(62) Divisional of application: 15736411.8
(73) Proprietor: Valitacell Limited, Dublin (IE)
(72) Inventor: THOMPSON, Ben, Sheffield, South Yorkshire S8 8QJ (GB); JAMES, David, Sheffield, South Yorkshire S119LN (GB); CLIFFORD, Jerry, Tralee, Co. Kerry Leebrook (IE)
(74) Representative: Purdylucey Intellectual Property

(56) References cited:
- EP-A1- 0 957 365
- EP-B1- 0 222 341
- EP-B1- 0 957 365
- WO-A1-2009/078876
- WO-A1-96/13722
- WO-A2-97/39326
- JP-A- 2005 337 805
- US-A1- 2006 105 397
- US-A1- 2011 143 387
- WENDY A LEA ET AL: "Fluorescence polarization assays in small molecule screening", EXPERT OPINION ON DRUG DISCOVERY, vol. 6, no. 1, 1 January 2011 (2011-01-01), pages 17 - 32, XP055208631, ISSN: 1746-0441, DOI: 10.1517/17460441.2011.537322
- AOYAGI S ET AL: "Development of fluorescence change-based, reagent-less optic immunosensor", BIOSENSORS AND BIOELECTRONICS, ELSEVIER BV, NL, vol. 20, no. 8, 15 February 2005 (2005-02-15), pages 1680 - 1684, XP027619500, ISSN: 0956-5663, [retrieved on 20050215]
- SUXIA ZHANG ET AL: "Fluorescence polarisation immunoassay based on a monoclonal antibody for the detection of sulphamethazine in chicken muscle", INTERNATIONAL JOURNAL OF FOOD SCIENCE AND TECHNOLOGY, vol. 42, no. 1, 1 January 2007 (2007-01-01), pages 36 - 44, XP055161505, ISSN: 0950-5423, DOI: 10.1111/j.1365-2621.2006.01202.x
- WANG Z H ET AL: "Analysis of Sulfamethazine by Fluorescence Polarization Immunoassay", CHINESE JOURNAL OF ANALYTICAL CHEMISTRY,, vol. 35, no. 6, 1 June 2007 (2007-06-01), pages 819 - 824, XP022857853, ISSN: 1872-2040, [retrieved on 20070601], DOI: 10.1016/S1872-2040(07)60068-1
- "Current Protocols in Chemical Biology", 1 December 2009, JOHN WILEY & SONS, INC., Hoboken, NJ, USA, ISBN: 978-0-47-055927-7, article NATHAN J. MOERKE: "Fluorescence Polarization (FP) Assays for Monitoring Peptide-Protein or Nucleic Acid-Protein Binding", XP055208888, DOI: 10.1002/9780470559277.ch090102

## Description

### Introduction

Immunofluorescence is a technique used for light microscopy with a fluorescence microscope and is used primarily on microbiological samples. This technique uses the specificity of antibodies to their antigen to target fluorescent dyes to specific biomolecule targets within a cell, and therefore allows visualisation of the distribution of the target molecule through the sample. Immunofluorescence is a widely used example of immunostaining and is a specific example of immunohistochemistry that makes use of fluorophores to visualise the location of the antibodies. Examples of fluorescent probes and their uses are described in CN103642261, WO2013153687, JP2012224646, US2012296098, WO2012149180, EP2312317, US20110143387, JP2010037511 and US4937198.

Fluorescent polarisation immunoassays are described in Nielsen et al (Methods 22, 71-76, 2000), WO9613722, WO2009078876, EP0957365, WO2010141249, and US2006105397, and can be employed for the rapid and accurate detection of antibody or antigen. The assay is based on the principle that small molecules rotate faster than larger molecules in solution, and that the rotation rate can be determined by fluorescence polarisation. When an antibody in a sample is incubated with a specific antigen for the antibody that is labelled with a fluorescent probe, the presence of antibody-antigen complex in the sample can be detected by means of fluorescence polarisation. A problem with this technique is that for detection of a given antibody, a specific antigen for that antibody is required which makes the assay expensive for many companies. A further problem with this technique is that is not suitable for quantitative detection of antibody in a sample.

Japanese Patent Application No: 2005-337805 describes a method of measuring antibodies or antigens using fluorescent polarisation that in one embodiment employs a conjugate of a recombinant generic antibody-binding protein (Protein A) and a short lifetime fluorescent dye (Alexa 647) for quantitative measurement of human IgG. This Japanese document describes the use of fluorescent labelled protein A at a concentration of either 1 or 10 nM, which equates to 0.00005 or 0.0005 mg/ml respectively. This protein A is expected to bind to the antibody at a ratio of 1:1, and as their molecular weights respectively are 50,000 Da and 150,000 Da, this means binding at a 1:3 weight ratio. Thus 1 mg of protein A would bind 3 mg of antibody and would thus be "saturated" meaning any increase in antibody concentration from this should not cause an increase in the polarisation value as no more binding would occur. Thus, 1 and 10nM of protein A would have an FP signal that would saturate (i.e. not increase any more from specific binding) at an IgG concentration of 0.000015 and 0.00015 mg/ml of antibody concentration. This clearly does not occur in the figures presented in the Japanese patent. The only significant increases occur far after this saturation limit, where any change in polarisation is not due to specific binding of the protein A to the antibody, but to artifactual effects such as increases in viscosity as antibody concentration increases. Thus, the data presented in this document indicates that in practice the invention does not work.

JP 2005 337805 A discloses a method for determining antibody concentration in a liquid sample comprising the steps of: incubating in a reaction chamber the liquid sample with an antibody-binding probe comprising a generic antibody binding protein (such as protein A and protein G) conjugated to a fluorescent probe to provide a reaction mixture; assaying the reaction mixture in the reaction chamber for fluorescence polarisation to detect a change in polarisation between excitation and emission light; and correlating the change in polarisation with antibody titre of the sample.

WO 96/13722 A1 discloses a method for determining antibody concentration and/or titre in a liquid sample comprising the steps of: incubating in a reaction chamber the liquid sample with an antibody-binding probe comprising an antibody binding protein conjugated to a fluorescent probe (an asymmetric metal-ligand complex conjugated to a molecule (such as antibody or lgG) which specifically binds said analyte) to provide a reaction mixture; assaying the reaction mixture in the reaction chamber for fluorescence polarisation to detect a change in polarisation between excitation and emission light; and correlating the change in polarisation with antibody titre of the sample. WO 96/13722 A1 also teaches the use of long life fluorescent dye.

WO 2009/078876 A1 teaches a high-throughput titre determining method using fluorescence polarisation detection.

Wendy A Lea et al. (Fluorescence polarization assays in small molecule screening. Expert Opin Drug Discov. 2011 Jan;6(1):17-32) teaches that the size of the binding probe/ligand and the life of the chosen fluorescent dye are critical characteristic influencing the strength of the measured signal. Wendy A Lea *et al*. also teaches the possibility of using generic antibody-binding probes and the use of peptides (truncated biding partner) conjugated to fluorescent dye in order to get higher sensitivity/signal.

Nathan J. Moerke (Fluorescence Polarization (FP) Assays for Monitoring Peptide-Protein or Nucleic Acid-Protein Binding. Current Protocols in Chemical Biology, 1: 1-15 (2009)) teaches "The key parameters for the fluorophore are the quantum yield and the fluorescence excited state lifetime. The fluorescence lifetime has a strong effect on the dependence of fluorescence polarization on molecular weight (Owicki, 2000). Fluorophores with lifetimes around 4 nsec, such as fluorescein and the rhodamines, provide a good separation in FP between small ligands and proteins of 10 kDa and higher".

It is an object of the invention to overcome at least one of the above-referenced problems.

### Brief Description of the Invention

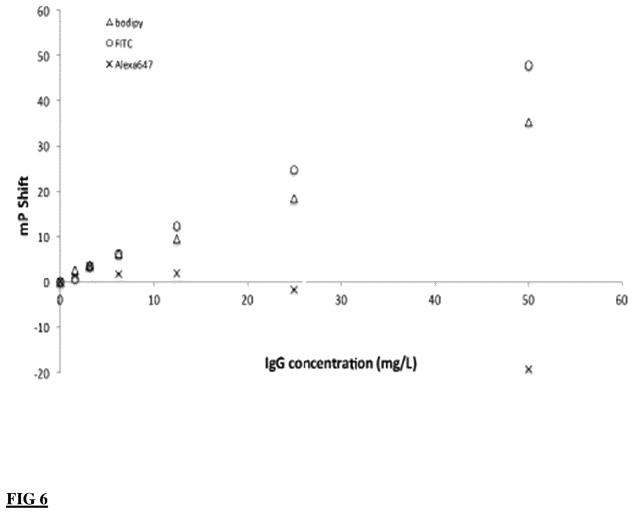

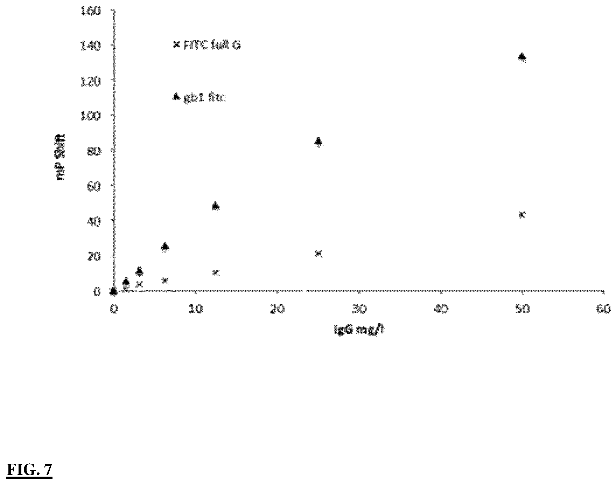

The invention is based on the finding that accurate and sensitive antibody concentration or titre in a sample can be determined using fluorescence polarisation and a tracer comprising a long life fluorescent dye conjugated to a generic immunoglobulin binding protein such as protein A, Protein G, Protein A/G, or Protein L (hereafter "generic antibody-binding protein"),. These proteins bind to the constant region of all immunoglobulin G (IgG) molecules, and therefore can be used in an assay for determining the concentration of any IgG molecule. Figure 6 demonstrates the effect of using a long lifetime dye compared with a short lifetime dye. In addition, the Applicant has surprisingly discovered that use of a truncanted generic antibody-binding protein, having a part of the protein removed (for example, one or more of the antibody-binding or non-antibody-binding sites removed), provides for a further increase in sensitivity. Figure 7 demonstrates the effect of using a highly truncated IgG binding protein (mw 7kD) compared with a partially truncated protein (mw 29kD).

In a first aspect, the invention provides a method for determining antibody titre in a liquid sample comprising the steps of:
incubating in a reaction chamber the liquid sample with an antibody-binding probe comprising a generic antibody binding protein conjugated to a fluorescent dye, typically a long lifetime fluorescent dye, to provide a reaction mixture;
assaying the reaction mixture in the reaction chamber for fluorescence polarisation to detect a change in polarisation between excitation and emission light; and
correlating the change in polarisation with antibody titre of the sample.

In embodiments employing a long life fluorescent dye, the long-life fluorescent dye preferably has a fluorescence lifetime of at least 4ns. Preferably, the long-life fluorescent dye has a fluorescence lifetime of at least 5ns. Preferably, the long-life fluorescent dye has a fluorescence lifetime of at least 6ns. Preferably, the long-life fluorescent dye has a fluorescence lifetime of at least 7ns. Preferably, the long-life fluorescent dye has a fluorescence lifetime of at least 8ns. Preferably, the long-life fluorescent dye has a fluorescence lifetime of at least 9ns. Preferably, the long-life fluorescent dye has a fluorescence lifetime of at least 10ns. Preferably, the long-life fluorescent dye has a fluorescence lifetime of at least 15ns. Preferably, the long-life fluorescent dye has a fluorescence lifetime of at least 20ns.

Preferably, the fluorescent dye is FITC. Preferably, the fluorescent dye is selected from FITC and a long life fluorescent probe having a lifetime of at least 5ns.

Preferably, the generic antibody binding protein is Protein A or Protein G.

Preferably, the generic antibody binding protein is a truncated generic antibody-binding protein.

Preferably, the sample is a cell culture fluid.

Preferably, the cell is a eukaryotic or prokaryotic producer cell.

Preferably, the reaction chamber is a well of a multiwell plate.

Preferably, the generic antibody binding protein (i.e. generic IgG binding protein) has a molecular weight of not more than 15kD and the fluorescent dye has a lifetime of at least 5 ns (ideally at least 10 or 15 ns). More preferably, the generic antibody binding protein (i.e. generic IgG binding protein) has a molecular weight of not more than 10kD and the long life fluorescent dye has a lifetime of at least 5 ns (ideally at least 10 or 15 ns). Ideally, the generic antibody binding protein (i.e. generic IgG binding protein) has a molecular weight of not more than 5kD and the long life fluorescent dye has a lifetime of at least 5 ns (ideally at least 10 or 15 ns).

In a second aspect the invention relates to a rapid, high-throughput, method of determining antibody titre in a plurality of cell culture samples comprising antibody producing cells (typically IgG producing cells, ideally monoclonal IgG producing cells), the method comprising the steps of:
incubating each cell culture sample in an individual well of a microtitre plate along with an antibody-binding probe comprising a generic antibody binding protein (i.e. generic IgG binding protein) conjugated to a long life fluorescent dye to provide a plurality of reaction mixtures;
assaying the reaction mixtures in the wells for fluorescence polarisation to detect a change in polarisation between excitation and emission light; and
correlating the change in polarisation with antibody titre of the cell culture samples.

In one embodiment, the plurality of cell culture samples comprises a panel of clonal producer cells. Thus, the invention also relates to a rapid, high-throughput, method of determining antibody titre in a panel of clonal producer cells.

Typically, the method employs a fluorescence polarisation analyser capable of performing a fluorescence polarisation assay on a plurality of wells of the microtitre plate simultaneously.

The invention also provides a kit suitable for performing the method the method of the invention and comprising (a) an antibody-binding probe comprising a generic antibody-binding protein conjugated to a fluorescent dye (preferably a long life fluorescent dye) and (b) a fluorescence polarisation analyser.

Preferably the generic antibody-binding protein is truncated.

Preferably the generic antibody-binding protein is an IgG-binding protein.

Preferably the IgG-binding protein is Protein G.

Preferably, the generic antibody binding protein comprises a truncated Protein G having a molecular weight of less than 15kD or 10kD.

Preferably, the fluorescent dye is FITC or has a fluorescence lifetime of at least 5ns.

The invention also relates to a conjugate of a fluorescent dye and a generic antibody binding protein.

In one embodiment, the generic antibody binding protein is truncated.

In one embodiment, the generic antibody binding protein has a molecular weight of less that 30kD. In one embodiment, the generic antibody binding protein has a molecular weight of less that 25kD. In one embodiment, the generic antibody binding protein has a molecular weight of less that 20kD. In one embodiment, the generic antibody binding protein has a molecular weight of less that 15kD. In one embodiment, the generic antibody binding protein has a molecular weight of less that 10kD. In one embodiment, the generic antibody binding protein has a molecular weight of less that 8kD. In one embodiment, the generic antibody binding protein has a molecular weight of less that 5kD.

In one embodiment, the fluorescent dye is a long life fluorescent dye. In one embodiment, the fluorescent dye has a lifetime of at least 4ns. In one embodiment, the fluorescent dye has a lifetime of at least 5ns. In one embodiment, the fluorescent dye is FITC or has a lifetime of at least 4ns.

Typically, the method of capable of accurately determining antibody concentration concentration in the range of 1-30 mg/L, preferably 1-40 mg/L, and ideally across 1-50 mg/L.

### Brief Description of the Figures

**Figure 1****:** Effect of fluorescence polarization of Dansyl labeled protein A, in the presence of IgG.
**Figure 2****:** Effect of fluorescence polarization of FITC labeled protein G, in the presence of IgG.
**Figure 3****:** Effect of fluorescence polarization of FITC labeled protein G, in the presence of IgG diluted in CD-CHO media.
**Figure 4****:** Effect of fluorescence polarization of FITC labeled protein G, in the presence of IgG diluted in water.
**Figure 5****:** Effect of fluorescence polarization of FITC labeled protein G, in the presence of unlabeled protein A diluted in water.
**Figure 6****:** Effect of fluorescence polarization on three different tracers in the presence of different concentrations of IgG diluted in CD-CHO. The tracers are conjugates of Protein G coupled to (1) BIODOPY (lifetime 5ns), (2) FITC (lifetime 4ns) and (3) Alexa647 (lifetime 1ns). The short lifetime dye (Alexa 647) provides a negligible signal, whereas the longer lifetime dyes provide excellent signals.
**Figure 7****:** Effect of fluorescence polarization on two different tracers in the presence of different concentrations of IgG diluted in CD-CHO. The tracers are conjugates of FITC (lifetime 4ns) coupled to (1) protein G (full G), and (2) truncated protein G (gb1) having two IgG binding sites removed. It is evident that use of a conjugate comprising highly truncated protein G gives the largest signal.

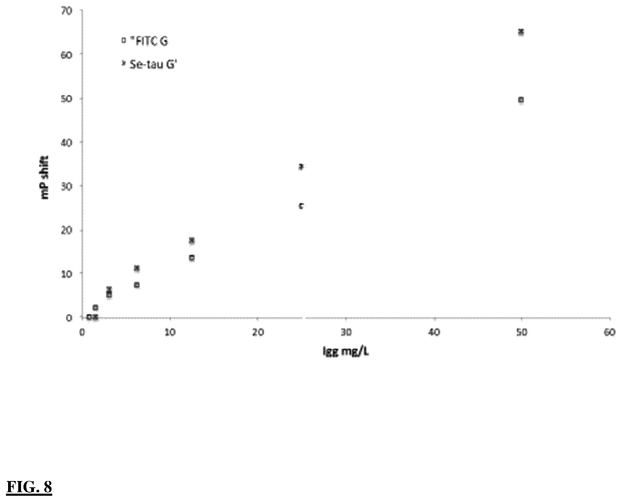

**Figure 8****:** Effect of fluorescence polarization on a different tracer in the presence of different concentrations of IgG diluted in CD-CHO. The tracer is a conjugate of Protein G coupled to a squaraine rotaxane dye which absorbs at 405nm and has a lifetime of around 9ns. It is evident that use of a longer lifetime dye provides an excellent signal.

### Detailed Description of the Invention

The invention relates to a method of determining, preferably quantitatively determining, the presence of an antibody, typically an IgG antibody, in a liquid sample, typically a cell culture sample, and ideally a cell culture sample comprising a monoclonal antibody producing cell. The method employs fluorescence polarisation analysis to detect binding between antibody, generally IgG antibody, and a generic antibody-binding probe. The antibody binding probe comprises a generic antibody binding protein, for example a generic IgG binding protein such as Protein A, G, L or A/G, conjugated to a fluorescent probe (dye), typically a long life fluorescent probe (dye) such as Dansyl. As the probe comprises a generic IgG binding tracer, it is capable of being used in assays for any IgG antibody, and is not specific to any particular IgG antibody. In use, the sample is incubated with the antibody binding probe, light emitted by the probe in the or each sample is analysed to detect fluorescence polarisation, and the level of fluorescence polarisation detected is correlated with antibody titre in the or each sample.

In this specification, the term "antibody" should be understood to mean an immunoglobulin, for example an IgG, IgA, IgE or IgM immunoglobulin in a monoclonal or polyclonal form, or a fragment thereof, in a humanised or non-humanised. In one embodiment, the antibody is an IgG molecule, preferably a monoclonal IgG molecule. In one embodiment, the antibody is a human antibody.

In this specification, the term "assaying the sample in the reaction chamber for fluorescence polarisation" should be understood to mean exciting the sample with plane polarised light at a wavelength corresponding to an excitation wavelength of the fluorescent dye, and detecting light intensity emitted by the fluorescent dye at an appropriate emission wavelength both in two planes, one of which is parallel to the plane of the excitation plane and one of which is perpendicular to the plane of the excitation light. In one embodiment, the excitation plane is vertical or horizontal and the emitted light is detected in vertical and horizontal planes. The degree to which the emission intensity moves from the excitation plane (i.e. vertical) to a perpendicular plane (i.e. horizontal) - i.e. the change in polarisation between excitation and emission light - is a function of the degree of rotation of the fluorescent dye. When the probe-labelled antibody binding protein is bound to antibody, the complex will rotate slower than the probe labelled antibody binding protein, resulting in an increase in polarisation of emitted light.

The term "correlating the change in polarisation with antibody concentration" should be understood to mean calculating the antibody concentration of the sample based on the change in polarisation of light emitted by the fluorescent probe. Methods for calculating the antibody concentration include inferring the antibody concentration from a standard curve of known concentrations of antibody or by calculating dissociation constants and necessary parameters, and inferring concentration from first principles. Competitive binding assays may also be employed to calculate antibody concentration.

In this specification, the term "rapid, high-throughput" should be understood to mean that the method can be carried out in 1 hours or less, preferably less than 30 minutes.

The term "antibody titer" or "antibody concentration" as used herein refers to the amount of antibody, generally a recombinant antibody, and ideally a recombinant monoclonal antibody, present in the sample at a given time point. Typically, the sample is a cell culture sample.

Preferably, the sample is supernatant derived from a cell culture sample. The titer or concentration may be quantified in absolute or relative terms. Generally titre or concentration is referred to as weight of product per volume of culture - grams per litre (g/L) is a common metric.

The term "sample" as used herein should be understood to mean a liquid sample, for example a cell culture sample or supernatant derived from a cell culture sample. Preferably, the cells are producer cells (i.e. cells genetically modified to produce a recombinant protein), for example prokaryotic producer cells (i.e. E. Coli) or eukaryotic producer cells (i.e. CHO cells). Examples of both prokaryothic and eukaryotic producer cells will be known to a person skilled in the art. The sample may also be blood or a blood derivative, for example plasma or serum. Preferably, cells are removed from the sample prior to determining antibody concentration.

The term "generic antibody binding protein" should be understood to mean a protein, peptide, polypeptide, or non-protein ligand such as an aptamer that binds to a constant region of an antibody, for example an IgG, IgA, IgE or IgM antibody. In a preferred embodiment, the generic antibody binding protein is a generic IgG binding protein that is typically specific for IgG antibodies, or an IgG binding variant or fragment thereof.. Examples of generic IgG binding proteins include Protein A, Protein G, Protein A/G, and Protein L, or antibody binding variants or fragments thereof (http://www.piercenet.com/method/antibody-igg-binding-proteins). In one embodiment, the generic antibody binding protein is a truncated generic antibody binding protein, preferably a protein normally comprising a plurality of homologous antibody binding domains and which is modified to remove one or more of the antibody binding domains. Preferably, the antibody binding protein is Protein G. Preferably, the antibody binding protein is truncated Protein G. In one embodiment, the protein is a truncated Protein G obtained from Sigma Aldrich (Paisley, UK). In one embodiment, the generic antibody binding protein has a molecular weight of less that 30kD. In one embodiment, the generic antibody binding protein has a molecular weight of less that 25kD. In one embodiment, the generic antibody binding protein has a molecular weight of less that 20kD. In one embodiment, the generic antibody binding protein has a molecular weight of less that 15kD. In one embodiment, the generic antibody binding protein has a molecular weight of less that 10kD. In one embodiment, the generic antibody binding protein has a molecular weight of less that 8kD. In one embodiment, the generic antibody binding protein has a molecular weight of less that 5kD. In one embodiment, the generic antibody binding protein is an aptamer (i.e. a DNA aptamer). In one embodiment, the generic antibody binding protein is a fragment of an antibody, for example a single chain variable fragment.

In this specification, the term "truncated generic antibody-binding protein" should be understood to mean a generic antibody binding protein that is modified to remove part of the protein. Reducing the MW of the generic antibody binding protein has been found to increase the signal of the conjugate in Fluorescent polarization assays. In one embodiment, at least one of the antibody binding regions is removed. In one embodiment, a part of the protein not involved in antibody binding is removed. The term typically includes truncated protein and fragments of generic antibody binding proteins. In one embodiment, the fragment includes at least one antibody-binding domain (for example gb1 in Example 7). In the case of truncated Protein G, one or two of the three antibody binding regions may be removed. In the case of truncated Protein A, one, two or three of the four antibody binding regions may be removed. Examples of truncated proteins are recombinant Protein G from Sigma, Abcam and Molecular Probes, which have a molecular weight of approximately 20kD and are modified to have at least one IgG binding domain removed. Another example is a peptide gb1 (see Example 7 below) which is a fragment of Protein G and a His-tag having a molecular weight 7kD. Fragments of truncated Protein G are described in Wilton et al (Proteins: Structure, Function and Bioinformatics, Vol. 71, Issue 3, P1432-1440). In one embodiment, the truncated generic antibody binding protein is truncated Protein G having a MW of less than 30kD. In one embodiment, the truncated generic antibody binding protein is truncated Protein G having a MW of less than 25kD. In one embodiment, the truncated generic antibody binding protein is truncated Protein G having a MW of less than 20kD. In one embodiment, the truncated generic antibody binding protein is truncated Protein G having a MW of less than 15kD. In one embodiment, the truncated generic antibody binding protein is truncated Protein G having a MW of less than 10kD. In one embodiment, the truncated generic antibody binding protein is truncated Protein G having a MW of less than 8kD. In one embodiment, the truncated generic antibody binding protein is truncated Protein A having a MW of less than 55kD. In one embodiment, the truncated generic antibody binding protein is truncated Protein A having a MW of less than 50kD. In one embodiment, the truncated generic antibody binding protein is truncated Protein A having a MW of less than 40kD. In one embodiment, the truncated generic antibody binding protein is truncated Protein A having a MW of less than 30kD. In one embodiment, the truncated generic antibody binding protein is truncated Protein A having a MW of less than 20kD. In one embodiment, the truncated generic antibody binding protein is truncated Protein A having a MW of less than 15kD. In one embodiment, the truncated generic antibody binding protein is truncated Protein A having a MW of less than 10kD.

The term "variant" is also intended to include mimics (i.e. peptide mimics) and chemical derivatives of generic antibody binding protein, i.e. where one or more residues of the generic antibody binding protein is chemically derivatized by reaction of a functional side group. Also included within the term variant are generic antibody binding proteins are in which naturally occurring amino acid residues are replaced with amino acid analogues. Examples of generic antibody binding protein variants are described in Dinon et al (J. Mol. Recognit. 2011 Nov-Dec; 24(6)).

Proteins and polypeptides (including variants and fragments thereof) of and for use in the invention may be generated wholly or partly by chemical synthesis or by expression from nucleic acid. The proteins and peptides of and for use in the present invention can be readily prepared according to well-established, standard liquid or, preferably, solid-phase peptide synthesis methods known in the art (see, for example, J. M. Stewart and J. D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984).

In this specification, the term "fluorescent probe" or "fluorescent dye" or "fluorophore" should be understood to mean a fluorescent chemical that can re-emit light upon light excitation. Examples of fluorescent probes will be known to those skilled in the art, and include fluorescent proteins such as GFP, YFP and RFP, and non-protein organic fluorophores including tetrapyrrole derivatives, pyrene derivatives, xanthene derivatives, and cyanine derivatives.

In this specification, the term "long life fluorescent dye" should be understood to mean a fluorescent dye having a fluoresence lifetime of at least 3, 4, 5, 6, 7, 8, 9, 10, 12, 14, 16, 18 or 20 nanoseconds. According to the invention, the fluorescent dye has a fluoresence lifetime of at least 4ns.

Preferably, the fluorescent dye has a fluoresence lifetime of at least 5ns. Preferably, the fluorescent dye has a fluoresence lifetime of 5-100 ns, 5-50 ns, 5-40 ns, 5-30 ns or 5-25 ns, typically 10-100 ns, 10-50 ns, 10-40 ns, 10-30 ns or 5-25 ns, and ideally 15-100 ns, 15-50 ns, 15-40 ns, 15-30 ns or 15-25 ns. Preferably, the fluorescent dye has a fluoresence lifetime of 4-100 ns, 4-50 ns, 4-40 ns, 4-30 ns or 4-25 ns, typically 10-100 ns, 10-50 ns, 10-40 ns, 10-30 ns or 5-25 ns, and ideally 15-100 ns, 15-50 ns, 15-40 ns, 15-30 ns or 15-25 ns. Examples of fluorescent dyes, including long life fluorescent dyes, are provided in the Table below.

| Fluorophore | Lifetime [ns] | Solvent | Excitation max [nm] | Emission max [nm] |
|---|---|---|---|---|
| 5-Hydroxytryptamine | | | 370-415 | 520-540 |
| ATTO 565 | 3.4 | Water | 561 | 585 |
| ATTO 655 | 3.6 | Water | 655 | 690 |
| Acridine Orange | 2 | PB pH 7.8 | 500 | 530 |
| Acridine Yellow | | | 470 | 550 |
| Alexa Fluor 488 | 4.1 | PB pH 7.4 | 494 | 519 |
| Alexa Fluor 532 | | | 530 | 555 |
| Alexa Fluor 546 | 4 | PB pH 7.4 | 554 | 570 |
| Alexa Fluor 633 | 3.2 | Water | 621 | 639 |
| Alexa Fluor 647 | 1 | Water | 651 | 672 |
| Alexa Fluor 680 | 1.2 | PB pH 7.5 | 682 | 707 |
| BODIPY 500/510 | | | 508 | 515 |
| BODIPY 530/550 | | | 534 | 554 |
| BODIPY FL | 5.7 | Methanol | 502 | 510 |
| BODIPY TR-X | 5.4 | Methanol | 588 | 616 |
| Cascade Blue | | | 375 | 410 |
| Coumarin 6 | 2.5 | Ethanol | 460 | 505 |
| CY2 | | | 489 | 506 |
| CY3B | 2.8 | PBS | 558 | 572 |
| CY3 | 0.3 | PBS | 548 | 562 |
| CY3.5 | 0.5 | PBS | 581 | 596 |
| CY5 | 1 | PBS | 646 | 664 |
| CY5.5 | 1 | PBS | 675 | 694 |
| Dansyl | 10 | | 340 | 520 |
| DAPI | 0.16 | TRIS/EDTA | 341 | 496 |
| DAPI + ssDNA | 1.88 | TRIS/EDTA | 358 | 456 |
| DAPI + dsDNA | 2.2 | TRIS/EDTA | 356 | 455 |
| DPH | | | 354 | 430 |
| Erythrosin | | | 529 | 554 |
| Ethidium Bromide - no DNA | 1.6 | TRIS/EDTA | 510 | 595 |
| Ethidium Bromide + ssDNA | 25.1 | TRIS/EDTA | 520 | 610 |
| Ethidium Bromide + dsDNA | 28.3 | TRIS/EDTA | 520 | 608 |
| FITC | 4.1 | PB pH 7.8 | 494 | 518 |
| Fluorescein | 4 | PB pH 7.5 | 495 | 517 |
| FURA-2 | | | 340-380 | 500-530 |
| GFP | 3.2 | Buffer pH 8 | 498 | 516 |
| Hoechst 33258 - no DNA | 0.2 | TRIS/EDTA | 337 | 508 |
| Hoechst 33258 + ssDNA | 1.22 | TRIS/EDTA | 349 | 466 |
| Hoechst 33258 + dsDNA | 1.94 | TRIS/EDTA | 349 | 458 |
| Hoechst 33342 - no DNA | 0.35 | TRIS/EDTA | 336 | 471 |
| Hoechst 33342 + ssDNA | 1.05 | TRIS/EDTA | 350 | 436 |
| Hoechst 33342 + dsDNA | 2.21 | TRIS/EDTA | 350 | 456 |
| HPTS | 5.4 | PB pH 7.8 | 454 | 511 |
| Indocyanine Green | 0.52 | Water | 780 | 820 |
| Laurdan | | | 364 | 497 |
| Lucifer Yellow | 5.7 | Water | 428 | 535 |
| Nile Red | | | 485 | 525 |
| Oregon Green 488 | 4.1 | Buffer pH 9 | 493 | 520 |
| Oregon Green 500 | 2.18 | Buffer pH 2 | 503 | 522 |
| Oregon Green 514 | | | 511 | 530 |
| Prodan | 1.41 | Water | 361 | 498 |
| Pyrene | > 100 | Water | 341 | 376 |
| Rhodamine 101 | 4.32 | Water | 496 | 520 |
| Rhodamine 110 | 4 | Water | 505 | 534 |
| Rhodamine 123 | | | 505 | 534 |
| Rhodamine 6G | 4.08 | Water | 525 | 555 |
| Rhodamine B | 1.68 | Water | 562 | 583 |
| Ru(bpy)₃[PF₆]₂ | 600 | Water | 455 | 605 |
| Ru(bpy)₂(dcpby)[PF₆]₂ | 375 | Buffer pH 7 | 458 | 650 |
| SeTau-380-NHS | 32.5 | Water | 270 | 480 |
| SeTau-404-NHS | 9.3 | Water | 402 | 515 |
| SeTau-405-NHS | 9.3 | Water | 405 | 518 |
| | | | 340 | |
| SeTau-425-NHS | 26.2 | Water | 425 | 545 |
| SITS | | | 336 | 438 |
| SNARF | | | 480 | 600-650 |
| Stilbene SITS, SITA | | | 365 | 460 |
| Texas Red | 4.2 | Water | 589 | 615 |
| TOTO-1 | 2.2 | Water | 514 | 533 |
| YOYO-1 no DNA | 2.1 | TRIS/EDTA | 457 | 549 |
| YOYO-1 + ssDNA | 1.67 | TRIS/EDTA | 490 | 510 |
| YOYO-1 + dsDNA | 2.3 | TRIS/EDTA | 490 | 507 |
| YOYO-3 | | | 612 | 631 |

The term "producer cell" refers to a cell that is employed to generate a specific desired protein. Generally, the cell is genetically modifed to include one or multiple copies of a transgene encoding the desired protein which is generally under the control of a specific promotor. Thus, the specific protein is usually a recombinant protein. Producer cells are well known in the art, and include for example Chinese hamster ovary (CHO) cells or baby hamster kidney (BHK) cells. Ideally, the producer cell is a CHO cell. Typically, the producer cell is a monoclonal antibody producer cell.

In this specification, the term "high-throughput" should be understood to mean a method in which a large number of samples, for example at least 20, 50, 90, 120, i.e. 20-500, can be assayed simultaneously.

In this specification, the term "multiwell plate" refers to a plate having a multiplicity of wells, for example at least 10, 15, 20, 30, 40, 50, 60, 70, 80, 90 100 or 384 wells. In one embodiment, the plate may be a solid, non-flexible plate and alternatively may be provided as a roll of flexible material.

In this specification, the term "panel of clonal producer cells" should be understood to mean a panel of clonal producer cell populations derived from a single cell line, and comprising from 2 to 500 or more clonal producer cell populations. Methods for generating panels of clonal producer cells are well known to a person skilled in the art, and described in Production of recombinant protein therapeutics in cultivated mammalian cells (2004) ,Wurm, Florian M, New York, NY, Nature Biotechnology 22 (2004), S. 1393-1398. Typically, the panel of clonal producer cell populations include from 10-500, 20-500, 30-500, 40-500, 50-500, 60-500, 70-500, 80-500, 90-500 or 100-500 clonal cell populations. Typically, the panel of clonal producer cell populations include from 100-500, 100-400, 150-400, 150-350 clonal cell populations.

In this specification, the term "fluoresence polarisation analyser" should be understood to mean mean a machine capable of receiving a sample and exciting the sample with plane polarised light at a wavelength corresponding to an excitation wavelength of a fluorescent dye, detecting light intensity emitted by the fluorescent dye at an appropriate emission wavelength in two planes, one parallel and one perpendicular to the emission plane, and typically determining the change in polarisation between excitation and emission light. Typically, the machine is capable of receiving and reading a multiplicity of sample contained in a multiwall plate, typically in a high-throughput manner. Examples of fluorescence polarization analysers include BMG PheraStar, BMG ClarioStar, Tecan Infinite F500.

### Experimental

### Experimental Methods for evaluating quantitation of IgG using a fluorescently labeled generic IgG binding protein.

Dansyl protein A was created by the covalent attachment of Dansyl chloride (5-(dimethylamino)naphthalene-1-sulfonyl chloride) (Sigma, UK) to recombinant Protein A from staphylococcus aerueus (Sigma, UK).

FITC labeled truncated protein G was obtained from Sigma, UK.

Immunoglobulin G (Human, active) was obtained from Abcam (Cambridge, UK).

Fluorescence polarization measurements were performed using a BMG ClarioStar (Bucks, UK). Readings were performed in black half area 96 'non-binding surface' well plates (Coming, UK, product 3686). Target mP values for negative controls were 70 mP. mP values were calculated from blank subtracted fluorescence.

### Evaluation of Dansyl-labelled truncated protein A conjugate.

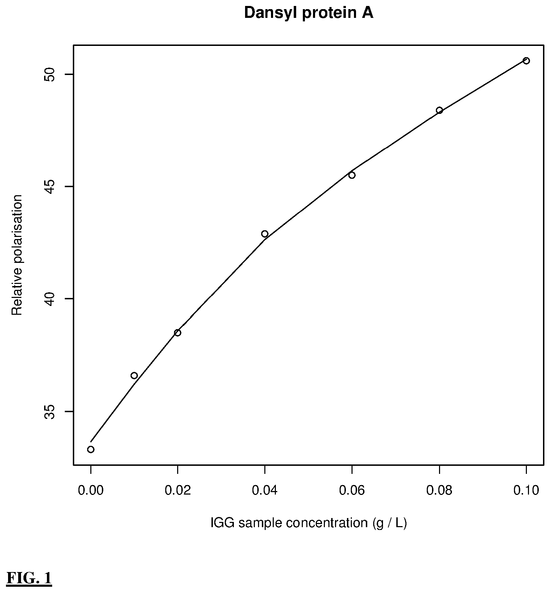

Here 25ul of 0.03 mg / mL of Dansyl protein A, diluted in water was mixed with 25 ul of full length human active IgG1 diluted in water. The samples were mixed and read immediately. Figure 1 shows the effect of fluorescence polarization of Dansyl labeled protein A, in the presence of IgG. It is apparent that using this technique it is possible to accurately quantify IgG over this range. There is a clear dose response change in mP with change in concentration of IgG.

### Evaluation of FITC truncated protein G conjugate.

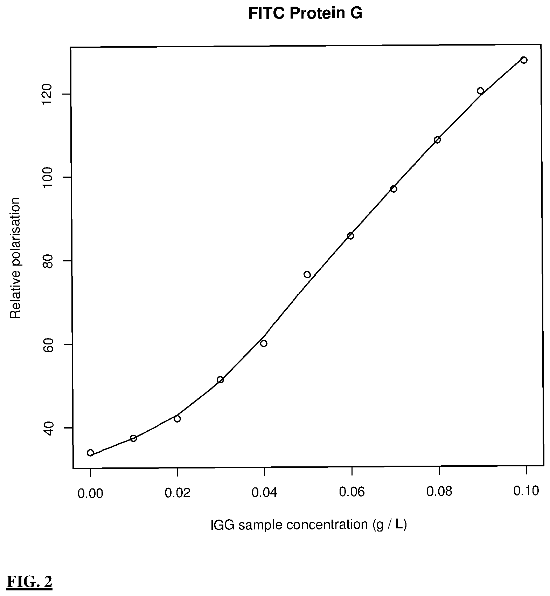

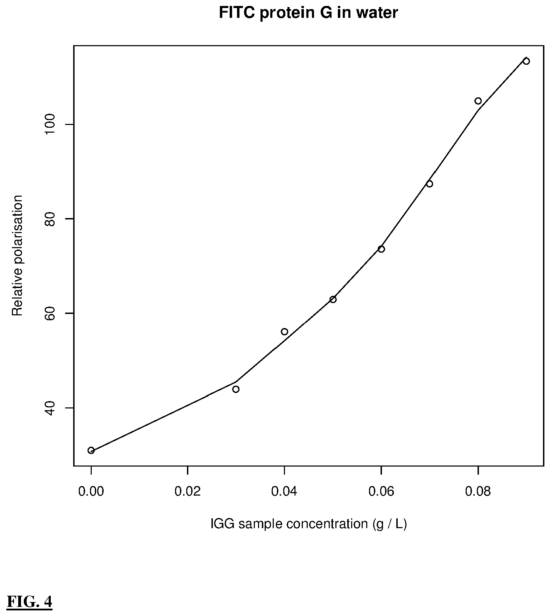

This technique was repeated using commercially available FITC conjugated truncated protein G (Sigma etc). Here 25ul of 0.03 mg / mL of FITC protein G diluted in water was mixed with 25 ul of full length human active IgG1 (Abcam, etc) diluted in water to various concentrations. Figures 2 and 4 show the effect of fluorescence polarization of FITC labeled protein G, in the presence of IgG. It is evident that this approach can be used to give precise quantitation of IgG over the range observed. The resolution is clearly high and differences of 0.01 g/L can be easy observed. This demonstrates that the approach can be used using a shorter life fluorophore such as FITC (Fluorescein isothiocyanate).

### Comparison of short lifetime fluorophore with long lifetime fluorophore.

The technique was repeated using three probes, namely (1) FITC-truncated protein G, (2) BIODOPY-truncated protein G and (3) Alexa647-truncated protein G. Here 30 ul of 0.02 mg / mL of conjugate diluted in protein G binding buffer (sigma, UK) was mixed with 30 ul of full length human active IgG1 (Abcam, etc) diluted in CD-CHO to various concentrations. Figure 6 show the effect of fluorescence polarization of the three conjugates in the presence of IgG. It is evident that this approach can be used to give precise quantitation of IgG over the range observed. It is also evident that the signal achieved with the conjugates with the longer life probe (FITC = 4ns, BIODOPY = 5ns) was significantly better than that achieved with the short lifetime probe (Alexa647 = 1ns).

### Comparison of truncated Protein G with Protein G fragment

The technique was repeated using two probes, namely (1) FITC conjugated to truncated protein G and (2). FITC conjugated to a fragment of Protein G containing a single IgG binding domain (gb1). Here 30 ul of 0.02 mg / mL of each conjugate diluted in protein G binding buffer (sigma, UK) was mixed with 25 ul of full length human active IgG1 (Abcam, etc) diluted in CD-CHO to various concentrations. Figure 7 show the effect of fluorescence polarization of the two conjugates in the presence of IgG. It is evident that the signal achieved with the Protein G fragment (<10kD) is significantly better than that achieved with the truncated Protein G (20kD).

### Evaluation of a squarene rotoxane dye- truncated protein G conjugate.

This technique was repeated using a squaraine rotoxene conjugated truncated protein G. Here 30ul of 0.02 mg / mL of squarene rotoxene protein G diluted in protein G binding buffer (sigma, UK) was mixed with 30 ul of full length human active IgG1 (Abcam, etc) diluted in CD-CHO media to various concentrations. Figure 7 shows the effect of fluorescence polarization of the conjugate, in the presence of IgG. It is evident that this approach can be used to give precise quantitation of IgG over the range observed. Squaraine rotoxane dyes are described in http://chem.isc.kharkov.com/dep9/research-achievements.

### Evaluation of fluorescence polarization approach to quantitate IgG in mammalian culture media.

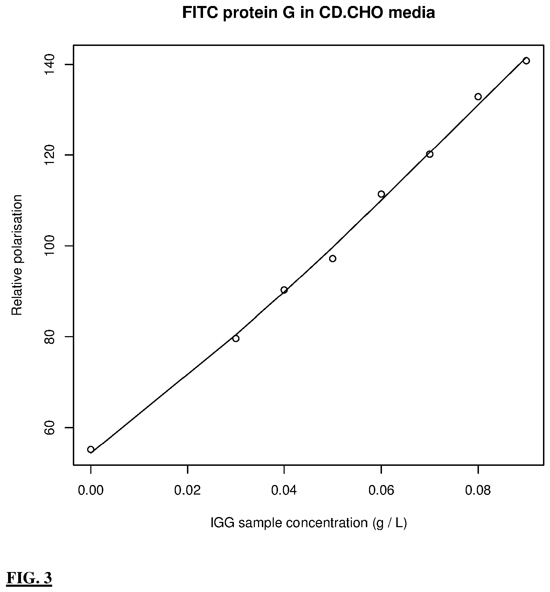

The effect of IgG sample diluted in culture media was evaluated. Here 25ul of 0.03 mg / mL of FITC protein G diluted in water was mixed with 25 ul of various dilutions of full length human active IgG1 (Abcam, etc) diluted in CD-CHO (Life Technologies, Paisley, UK). Figure 3 shows the effect of fluorescence polarization of FITC labeled protein G, in the presence of IgG diluted in CD-CHO media.

### Negative control evaluation of mP in the presence of a non-target protein.

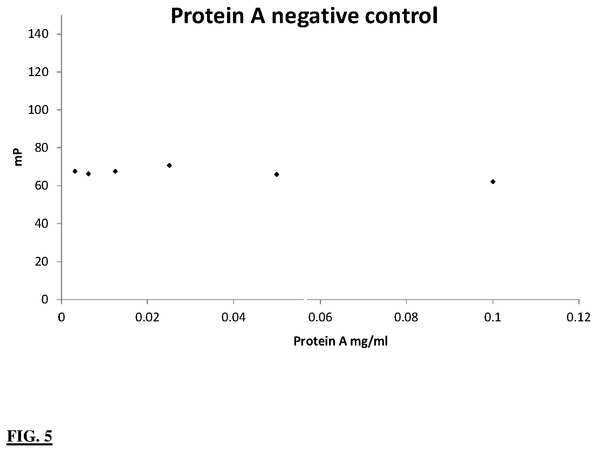

As a negative control, FITC protein G was added to various concentrations of unlabeled protein A at comparable concentrations to the IgG concentrations. Figure 5 shows the effect of fluorescence polarization of FITC labeled protein G, in the presence of unlabeled protein A diluted in water. Here it is apparent that there is no systematic change in polarization when a non-target protein is used.

The above results clearly demonstrate that the methods of the invention may be employed for the rapid, inexpensive and high-throughput quantitation of IgG antibody.

## Claims

1. A method for determining antibody concentration in a liquid sample comprising the steps of:
incubating in a reaction chamber the liquid sample with an antibody-binding probe comprising a generic antibody-binding protein conjugated to a fluorescent dye to provide a reaction mixture;
assaying the reaction mixture in the reaction chamber for fluorescence polarisation to detect a change in polarisation between excitation and emission light; and
correlating the change in polarisation with antibody titre of the sample,
**characterised in that** the fluorescent dye is a long life fluorescent dye with a lifetime of at least 4ns and in which the generic antibody-binding protein is truncated Protein A having a molecular weight of less than 40kD, or less than 30kD, or less than 20kD, or less than 10kD.

2. A method as claimed in Claim 1 in which the long life fluorescent dye has a lifetime of at least 5ns.

3. A method as claimed in Claim 1 or Claim 2 in which the long life fluorescent dye is FITC or a fluorescent dye having a lifetime of at least 5ns.

4. A method as claimed in any preceding Claim, in which the reaction chamber is a well of a multiwell plate.

5. A method as claimed in any preceding Claim in which the sample is a cell culture fluid; and optionally from a biopharmaceutical producer cell culture.

6. A kit comprising (a) an antibody-binding probe comprising a truncated Protein A having a molecular weight of less than 40kD, or less than 30kD, or less than 20kD, or less than 10kD, conjugated to a long life fluorescent dye having a fluorescence lifetime of at least 4ns, and (b) a fluorescence polarisation analyser.

7. A kit as claimed in Claim 6 in which the antibody binding probe is FITC or a fluorescent probe having a fluorescence lifetime of at least 5ns.

8. A kit as claimed in Claim 7 including one or more multiwell plates.

9. A conjugate of a long life fluorescent dye having a fluorescence lifetime of at least 4ns and a truncated generic antibody binding protein, wherein the truncated generic antibody binding protein is truncated Protein A having a molecular weight of less than 40kD.

10. A conjugate according to Claim 9 in which the truncated generic antibody binding protein is truncated Protein A having a molecular weight of less than 30kD, or less than 20kD, or less than 10kD.

11. A conjugate according to Claim 9 or Claim 10 in which the fluorescent dye is FITC or is a fluorescent dye having a lifetime of at least 5ns.

## Patentansprüche

1. Verfahren zum Bestimmen einer Antikörperkonzentration in einer flüssigen Probe, umfassend die Schritte:
Inkubieren der flüssigen Probe in einer Reaktionskammer mit einer Antikörper-bindenden Sonde, die ein generisches Antikörper-bindendes Protein umfasst, das an einen Fluoreszenzfarbstoff konjugiert ist, um ein Reaktionsgemisch bereitzustellen;
Untersuchen des Reaktionsgemisches in der Reaktionskammer auf Fluoreszenzpolarisation, um eine Änderung der Polarisation zwischen Anregungs- und Emissionslicht zu erfassen; und
Korrelieren der Änderung der Polarisation mit einem Antikörpertiter der Probe, **dadurch gekennzeichnet, dass** der Fluoreszenzfarbstoff ein langlebiger Fluoreszenzfarbstoff mit einer Lebensdauer von mindestens 4 ns ist, und wobei das generische Antikörper-bindende Protein ein verkürztes Protein A mit einem Molekulargewicht von weniger als 40 kD, oder weniger als 30 kD, oder weniger als 20 kD, oder weniger als 10 kD ist.

2. Verfahren nach Anspruch 1, wobei der langlebige Fluoreszenzfarbstoff eine Lebensdauer von mindestens 5 ns aufweist.

3. Verfahren nach Anspruch 1 oder Anspruch 2, wobei der langlebige Fluoreszenzfarbstoff FITC oder ein Fluoreszenzfarbstoff mit einer Lebensdauer von mindestens 5 ns ist.

4. Verfahren nach einem vorstehenden Anspruch, wobei die Reaktionskammer eine Vertiefung einer Mehrfachvertiefungsplatte ist.

5. Verfahren nach einem vorstehenden Anspruch, wobei die Probe ein Zellkulturfluid ist; und optional aus einer Zellkultur eines biopharmazeutischen Produzenten stammt.

6. Kit, umfassend (a) eine Antikörper-bindende Sonde, die ein verkürztes Protein A mit einem Molekulargewicht von weniger als 40 kD, oder weniger als 30 kD, oder weniger als 20 kD, oder weniger als 10 kD umfasst, konjugiert an einen langlebigen Fluoreszenzfarbstoff mit einer Fluoreszenzlebensdauer von mindestens 4 ns, und (b) einen Fluoreszenzpolarisationsanalysator.

7. Kit nach Anspruch 6, wobei die Antikörper-bindende Sonde FITC oder eine fluoreszierende Sonde mit einer Fluoreszenzlebensdauer von mindestens 5 ns ist.

8. Kit nach Anspruch 7, einschließlich einer oder mehrerer Mehrfachvertiefungsplatten.

9. Konjugat aus einem langlebigen Fluoreszenzfarbstoff mit einer Fluoreszenzlebensdauer von mindestens 4 ns und einem verkürzten generischen Antikörper-bindenden Protein, wobei das verkürzte generische Antikörper-bindende Protein ein verkürztes Protein A mit einem Molekulargewicht von weniger als 40 kD ist.

10. Konjugat nach Anspruch 9, wobei das verkürzte generische Antikörper-bindende Protein ein verkürztes Protein A mit einem Molekulargewicht von weniger als 30 kDa, oder weniger als 20 kDa, oder weniger als 10 kDa ist.

11. Konjugat nach Anspruch 9 oder Anspruch 10, wobei der Fluoreszenzfarbstoff FITC ist oder ein Fluoreszenzfarbstoff mit einer Lebensdauer von mindestens 5 ns ist.

## Revendications

1. Procédé pour déterminer la concentration en anticorps dans un échantillon liquide comprenant les étapes suivantes :
l'incubation dans une chambre de réaction de l'échantillon liquide avec une sonde de liaison à un anticorps comprenant une protéine de liaison à l'anticorps générique conjuguée à un colorant fluorescent pour fournir un mélange réactionnel ;
le dosage du mélange réactionnel dans la chambre de réaction pour la polarisation de fluorescence pour détecter un changement de polarisation entre la lumière d'excitation et la lumière d'émission ; et
la corrélation du changement de polarisation avec le titre d'anticorps de l'échantillon, **caractérisé en ce que** le colorant fluorescent est un colorant fluorescent à longue durée de vie avec une durée de vie d'au moins 4 ns et dans lequel la protéine de liaison à l'anticorps générique est la protéine A tronquée présentant un poids moléculaire inférieur à 40 kD, ou inférieur à 30 kD, ou inférieur à 20 kD, ou inférieur à 10 kD.

2. Procédé selon la revendication 1, dans lequel le colorant fluorescent à longue durée de vie présente une durée de vie d'au moins 5 ns.

3. Procédé selon la revendication 1 ou la revendication 2 dans lequel le colorant fluorescent à longue durée de vie est le FITC ou un colorant fluorescent présentant une durée de vie d'au moins 5 ns.

4. Procédé selon une quelconque revendication précédente, dans lequel la chambre de réaction est un puits d'une plaque multipuits.

5. Procédé selon une quelconque revendication précédente dans lequel l'échantillon est un fluide de culture cellulaire ; et facultativement provenant d'une culture cellulaire de producteur biopharmaceutique.

6. Kit comprenant (a) une sonde de liaison à un anticorps comprenant une protéine A tronquée présentant un poids moléculaire inférieur à 40 kD, ou inférieur à 30 kD, ou inférieur à 20 kD, ou inférieur à 10 kD, conjuguée à un colorant fluorescent à longue durée de vie présentant une durée de vie de fluorescence d'au moins 4 ns, et (b) un analyseur de polarisation de fluorescence.

7. Kit selon la revendication 6 dans lequel la sonde de liaison à un anticorps est le FITC ou une sonde fluorescente présentant une durée de vie de fluorescence d'au moins 5 ns.

8. Kit selon la revendication 7 incluant une ou plusieurs plaques multipuits.

9. Conjugué d'un colorant fluorescent à longue durée de vie présentant une durée de vie de fluorescence d'au moins 4 ns et d'une protéine de liaison à l'anticorps générique tronquée, dans lequel la protéine de liaison à l'anticorps générique tronquée est une protéine A tronquée présentant un poids moléculaire inférieur à 40 kD.

10. Conjugué selon la revendication 9 dans lequel la protéine de liaison à l'anticorps générique tronquée est une protéine A tronquée présentant un poids moléculaire inférieur à 30 kD, ou inférieur à 20 kD, ou inférieur à 10 kD.

11. Conjugué selon la revendication 9 ou la revendication 10 dans lequel le colorant fluorescent est le FITC ou est un colorant fluorescent présentant une durée de vie d'au moins 5 ns.
